# EUROPÄISCHE PATENTSCHRIFT

(11) **EP 4 342 431 B1**
(45) Veröffentlichungstag und Bekanntmachung des Hinweises auf die Patenterteilung: **19.11.2025**
(21) Anmeldenummer: 23197849.5
(22) Anmeldetag: 18.09.2023
(51) Int. Cl.: A61F 2/30, A61F 2/38

(54) **KNIEGELENKIMPLANTATAUGMENT UND KNIEGELENKIMPLANTAT**
KNEE JOINT IMPLANT AUGMENT AND KNEE JOINT IMPLANT
IMPLANT D'ARTICULATION DU GENOU ET IMPLANT D'ARTICULATION DU GENOU

(30) Priorität: 21.09.2022 DE 102022124193
(43) Veröffentlichungstag der Anmeldung: 27.03.2024
(73) Patentinhaber: Aesculap AG, 78532 Tuttlingen (DE)
(72) Erfinder: HIRT, Martin, 78333 Stockach (DE); BOLLINGER, Arthur, 78532 Tuttlingen (DE)
(74) Vertreter: Winter, Brandl - Partnerschaft mbB

(56) Entgegenhaltungen:
- US-A- 5 458 637
- US-A1- 2009 265 011
- US-A1- 2014 222 155
- US-A1- 2014 277 539
- US-A1- 2020 281 731

## Beschreibung

### Technisches Gebiet

Die vorliegende Offenbarung betrifft ein Kniegelenkimplantataugment für ein Kniegelenkimplantat und ein Kniegelenkimplantat.

Kniegelenkprothesen oder -implantate oder Knieendoprothesen werden beim Ersatz des meist durch Kniearthrose zerstörten Kniegelenks eingesetzt. Dabei wird das Kniegelenk teilweise oder vollständig ersetzt.

Bekannte Kniegelenkimplantate weisen ein Femurelement, ein Tibiaelement und ein Inlay zwischen dem Femurelement und dem Tibiaelement auf. Das Inlay ist eine Gleitfläche, über die das Femurelement auf dem Tibiaelement abgleiten kann. Das Femurelement hat einen Schaft, um es fest mit dem Femur zu verbinden. Das Tibiaelement weist einen Tibiaschaft auf, mit dem das Tibiaelement fest mit der Tibia verbunden wird. Wenn ein großer Teil der Tibia entfernt wird, kann das dünne Tibiaelement den verlorenen Knochen nicht ersetzen. Deshalb wird zusätzlich ein sogenanntes Kniegelenkimplantataugment oder Knieimplantataugment verwendet. Das Kniegelenkimplantataugment ist dabei im Wesentlichen eine (flache) Platte, die als Abstandhalter zwischen der abgeschnittenen Tibia und dem Tibiaelement des Kniegelenkimplantats fungiert.

### Stand der Technik

Ein solches Kniegelenkimplantataugment ist beispielsweise aus der US 11 266 509 B2 bekannt. Dabei ist das Kniegelenkimplantataugment derart anatomisch gestaltet, dass es an die Form des abgeschnittenen Knochens angepasst ist. Eine distale oder untere Fläche des Kniegelenkimplantataugments ist dabei im Wesentlichen an eine Querschnittsform der abgeschnittenen Tibia angepasst. Ein Flächeninhalt der distalen Fläche ist kleiner als ein Flächeninhalt einer proximalen Fläche des Kniegelenkimplantataugments. Auch die EP 1 360 950 B1, US 2020 / 281 731 A1, US 2014 / 222 155 A1, US 2014 / 277 539 A1 und die US 5 458 637 A offenbaren solche Kniegelenkimplantataugmente. Aus der US 2009 / 265 011 A1 ist ein Kniegelenkimplantataugment mit einem rechten Winkel zwischen der Mantelfläche des Kniegelenkimplantataugments und der distalen und proximalen Fläche des Kniegelenkimplantataugments bekannt.

Bei bekannten Kniegelenkimplantataugmenten muss das gesamte Tibiaelement beim Anbringen in anteriore Richtung bzw. nach vorne zum Knie hin verschoben werden, um eine gute Auflage des Tibiaelements mit dem Kniegelenkimplantataugment auf der Tibia zu gewährleisten. In diesem Teil erweitert sich die Tibia nämlich in anteriore Richtung.

Dieses Verschieben in anteriore Richtung ist aber nachteilhaft. Zum einen resultiert daraus ein unerwünschter Überstand des Tibiaelements über den Knochen. Zum anderen geht durch die Umpositionierung während des Einsetzens des Implantats der Bezug zu einem Markkanal des Knochens verloren. Bei Revisionsoperationen werden häufig Schäfte verwendet, die im Optimalfall dem Markkanal des Knochens / der Tibia folgen sollten. Der Markkanal bildet somit eine mechanische Achse des Knochens / der Tibia. Durch das Verschieben nach vorne ist das Tibiaelement und insbesondere der Schaft des Tibiaelements nicht mehr entlang dem Markkanal / der mechanischen Achse der Tibia ausgerichtet. Der Fehler durch die Umpositionierung kann lediglich durch einstellbare Schäfte behoben werden. Jedoch sind die meisten Schäfte lediglich medial-lateral und nicht anterior-posterior einstellbar. Daher kann es zu Positionierungsfehlern der Tibia kommen. Somit können bekannte Kniegelenkimplantate unvorteilhaft eingesetzt werden.

Konkreter kann beim Einsetzens des Implantats ein Zielkonflikt auftreten. Wenn der Tibiaschaft an dem Markkanal der Tibia ausgerichtet wird, passt die Auflagefläche zwischen dem Implantat und besonders dem Kniegelenkimplantataugment nicht zu der abgeschnittenen Tibia. Wird das Augment aber an der Form der abgeschnittenen Tibia ausgerichtet, kann der Tibiaschaft von der mechanischen Achse des Markkanals abweichen. Dieser Zielkonflikt könnte durch Implantate mit einstellbaren Schäften gelöst werden. Die meisten Schäfte sind aber lediglich medial-lateral einstellbar. Ferner ist das Einstellen der Schäfte während der Operation nachteilhaft.

Kurzbeschreibung der Offenbarung

Die Aufgabe der vorliegenden Offenbarung ist es also, die Nachteile des Stands der Technik zu überkommen oder zumindest zu mindern, insbesondere ein Kniegelenkimplantataugment bereitzustellen, durch das ein Kniegelenkimplantat optimiert an der Tibia befestigt werden kann. Insbesondere soll ein Kniegelenkimplantataugment bereitgestellt werden, dass an die Form der Tibia angepasst ist. Ferner soll ein Kniegelenkimplantat bereitgestellt werden, das optimiert mit der Tibia verbunden werden kann.

Diese Aufgabe wird durch ein Kniegelenkimplantataugment gemäß dem Anspruch 1 und ein Kniegelenkimplantat gemäß dem Anspruch 7 gelöst. Vorteilhafte Weiterbildungen der vorliegenden Offenbarung sind Gegenstand der beigefügten Unteransprüche.

Die vorliegende Offenbarung betrifft ein Kniegelenkimplantataugment oder ein Spacer für ein Kniegelenkimplantat, das dazu vorbereitet und ausgebildet ist, in einem implantierten Zustand zwischen einem Kniegelenkimplantat und einer Tibia angeordnet zu sein. Das Kniegelenkimplantataugment weist eine erste Kontaktfläche, die ausgebildet ist, in dem implantierten Zustand zumindest abschnittsweise flächig an dem Kniegelenkimplantat aufzuliegen, eine der ersten Kontaktfläche gegenüberliegende zweite Kontaktfläche, die ausgebildet ist, in dem implantierten Zustand zumindest abschnittsweise flächig an der Tibia aufliegen zu können, und eine Mantelfläche oder Umfangsfläche auf, die einen Rand der ersten Kontaktfläche mit einem Rand der zweiten Kontaktfläche verbindet. Die Mantelfläche ist derart ausgebildet, dass ein Abschnitt der Mantelfläche / ein Mantelflächenabschnitt, welcher in dem implantierten Zustand des Kniegelenkimplantataugments patellar angeordnet ist, ausgehend von der ersten Kontaktfläche in einem Anstellwinkel größer als 0° bezüglich einer Achse in patellare Richtung angestellt ist, wobei die Achse senkrecht / orthogonal auf der ersten Kontaktfläche steht.

Das Kniegelenkimplantat kann das ursprüngliche Kniegelenk zumindest teilweise ersetzen und kann somit positionsbestimmt in den Körper eingesetzt oder einoperiert sein. Das Kniegelenkimplantataugment ist positionsbestimmt an dem Kniegelenkimplantat angeordnet und ist somit ebenfalls positionsbestimmt bezüglich des Kniegelenks bzw. des Körpers positioniert.

Positionsbestimmt bedeutet in diesem Zusammenhang, dass die Lage und Ausrichtung des Kniegelenkimplantataugments bezüglich des Kniegelenkimplantats durch geeignete Mittel an dem Kniegelenkimplantataugment oder an dem Kniegelenkimplantat oder durch ein Zusammenspiel von Mitteln an beiden Bauteilen definiert ist. Somit ist insbesondere definiert, in welche Richtung die einzelnen Abschnitte der Mantelfläche zeigen, wenn das gesamte Kniegelenkimplantat (bestimmungsgemäß) eingesetzt oder implantiert ist.

Die patellare Richtung ist in diesem Zusammenhang als die Richtung zu verstehen, die beim Menschen nach vorne zeigt, insbesondere in Richtung der Kniescheibe. Die patellare Ausrichtung ist als die anteriore Richtung des Körpers zu verstehen.

Die erste Kontaktfläche oder proximale Fläche ist in diesem Zusammenhang die Seite des Kniegelenkimplantataugments, die beim Menschen nach oben, also zum Oberkörper bzw. vom Knie aus zum Oberschenkel zeigt. Als die zweite Kontaktfläche oder kaudale oder distale Seite ist die Seite des Kniegelenkimplantataugments, die zum Fuß oder Unterschenkel zeigt, zu verstehen. Die erste Kontaktfläche liegt bei Montage des Kniegelenkimplantats also an dem Kniegelenkimplantat an, die zweite Kontaktfläche steht in Kontakt mit der Tibia.

Die Tibia erstreckt sich entlang ihrer Längsachse. Der Markkanal folgt im Wesentlichen der Längsachse der Tibia. Der patellare Mantelflächenabschnitt ist bezüglich der Längsachse der Tibia in dem Anstellwinkel ungleich 0° angestellt.

In anderen Worten ausgedrückt ist das Kniegelenkimplantataugment ein Augment oder einen Abstandshalter für ein Kniegelenkimplantat mit einer vorzugsweise flachen, annähernd u-oder hufeisenförmige Grundplatte, die eine erste (Grund-)Fläche, eine zweite (Grund-)Fläche, die der ersten (Grund-)Fläche gegenüberliegt und eine Mantelfläche aufweist, die die erste (Grund-)Fläche und zweite (Grund-)Fläche verbindet und begrenzt. Die Mantelfläche weist einen Abschnitt auf, der beim positionsbestimmten Anliegen des Kniegelenkimplantataugments an dem Kniegelenkimplantat und genauer an einer distalen Fläche eines Tibiaelements des Kniegelenkimplantats in die patellare oder anteriore Richtung zeigt.

Das Kniegelenkimplantataugment weist also an dem patellaren Mantelflächenabschnitt eine vorstehende Nase auf, wobei die Nase vorzugsweise an der distalen Seite des Mantelflächenabschnitts ausgebildet ist. Insgesamt ist der anteriore Abschnitt derart ausgebildet und angepasst, dass die zweite Kontaktfläche des Kniegelenkimplantataugments zumindest teilweise der Form der (abgeschnittenen) Tibia entspricht.

Die Achse kann ein Normalenvektor auf die erste Kontaktfläche sein. Da die erste Kontaktfläche und die zweite Kontaktfläche vorzugsweise parallel zueinander ausgebildet sind, kann die Achse auch ein Normalenvektor auf die erste Kontaktfläche sein. In dem implantierten Zustand des Kniegelenkimplantataugments kann die Achse parallel zu der Längsachse des Markkanals der Tibia ausgerichtet sein oder der Längsachse des Markkanals entsprechen.

Durch das offenbarungsgemäße Kniegelenkimplantataugment ist das Kniegelenkimplantat optimiert an die Form der Tibia angepasst. Dadurch hält das Kniegelenkimplantataugment besser an der Tibia bzw. die Auflage des Tibiaelements des Kniegelenkimplantats mit dem Kniegelenkimplantataugment an der Tibia ist besser gewährleistet. Insbesondere weist das Kniegelenkimplantataugment eine große Auflagefläche auf der abgeschnittenen Tibia auf. Somit muss das Tibiaelement bei der Operation nicht in die anteriore / patellare Richtung verschoben werden, um die Auflagefläche zwischen Kniegelenkimplantataugment und der Tibia zu vergrößern, und kann somit in der optimalen Position verbleiben. In der optimalen Position ist ein Schaft des Tibiaelements bezüglich des Markkanals der Tibia ausgerichtet. Das Tibiaelement kann somit besser an der Tibia befestigt und Positionierungsfehler der Tibia können vermieden werden. Insbesondere ist das Tibiaelement entlang der mechanischen Achse bzw. dem Markkanal der Tibia ausgerichtet.

Ferner ist die Herstellung des offenbarungsgemäßen Kniegelenkimplantataugments einfach und kostengünstig möglich.

Die Aufgabe der vorliegenden Offenbarung wird ferner durch eine Kniegelenkprothese / ein Kniegelenkimplantat gelöst. Das Kniegelenkimplantat weist ein Femurelement bzw. -implantat und ein Tibiaelement bzw. -implantat auf. Das Tibiaelement weist eine Grundplatte, einen Schaft, der sich von der Grundplatte in distale Richtung erstreckt und dafür angepasst ist, in die Tibia eingebracht zu werden, und vorzugsweise einen (Verstärkungs-)Flügel auf, der sich zwischen der Grundplatte und dem Schaft erstreckt. Ferner kann das Kniegelenkimplantat vorzugsweise ein Inlay / eine Gleitfläche / ein Einlegeteil aufweisen, das zwischen dem Femurelement und dem Tibiaelement angebracht ist und ein Abgleiten der beiden Elemente aufeinander ermöglicht. Das Kniegelenkimplantat weist ferner ein Kniegelenkimplantataugment mit einer ersten Kontaktfläche, einer der ersten Kontaktfläche gegenüberliegenden zweiten Kontaktfläche und einer Mantelfläche auf, die einen Rand der ersten Kontaktfläche mit einem Rand der zweiten Kontaktfläche verbindet. Die erste Kontaktfläche liegt zumindest abschnittsweise flächig an der Grundplatte an. Die zweite Kontaktfläche ist ausgebildet, zumindest abschnittsweise flächig an dem Knochen aufliegen zu können. Die Mantelfläche ist derart ausgebildet, dass ein Abschnitt der Mantelfläche, welcher in dem implantierten Zustand des Gelenkimplantats patellar angeordnet ist, in dem implantierten Zustand in einem Anstellwinkel größer als 0° bezüglich einer Längsachse des Schafts in patellare Richtung angestellt ist.

In dem implantierten Zustand des Kniegelenkimplantats erstreckt sich der Schaft des Tibiaelements in distale Richtung von der Grundplatte. Der Schaft ist ausgebildet, im implantierten Zustand in der Tibia versenkt / in die Tibia eingebracht zu werden. Dabei erstreckt sich die Längsachse entlang der Längsachse des Markkanals der Tibia.

Das Kniegelenkimplantat kann durch den angestellten patellaren Mantelflächenabschnitt besonders fest mit den Knochen verbunden werden und ist einfach einzusetzen bzw. einzuoperieren, da die Auflagefläche des Kniegelenkimplantats auf der Tibia besonders groß ist. Insbesondere muss das Kniegelenkimplantat und besonders das Tibialement beim Einsetzen nicht nach vorne verschoben werden. Das Kniegelenkimplantat ist somit beim Implantieren zur Längsachse der Tibia und der Längsachse des Markkanals ausgerichtet.

Das Kniegelenkimplantat ist vorzugsweise zwischen einem distalen Abschnitt des Femurs und einem proximalen Abschnitt der Tibia positioniert. Das Femurelement kann dabei mit dem Femur und das Tibiaelement mit der Tibia verbunden sein. Die Teilimplantate können dabei insbesondere über die jeweiligen Schäfte mit den entsprechenden Knochen verbunden sein.

Nach einem optionalen Aspekt der vorliegenden Offenbarung ist der patellare Mantelflächenabschnitt in dem implantierten Zustand des Kniegelenkimplantataugments in dem Anstellwinkel größer als 0° bezüglich einer Längsachse eines Markenkanals der Tibia angestellt. Insbesondere kann der patellare Mantelabschnitt eine Nase aufspannen, die über eine Grundfläche der ersten Kontaktfläche hinaussteht.

Das Kniegelenkimplantataugment kann annähernd die Form eines (schiefen) Zylinders oder eines Kegelstumpfs aufweisen. Die Achse kann dabei eine Längsachse des Zylinders und / oder des Kegelstumpfs darstellen.

Ein Winkel, der zwischen der ersten Kontaktfläche und dem patellaren Mantelflächenabschnitt aufgespannt ist, kann also ungleich 90° sein. Besonders bevorzugt ist der Winkel zwischen der ersten Kontaktfläche und dem patellaren (angestellten) Mantelflächenabschnitt in dem implantierten Zustand des Kniegelenkimplantataugments größer als 90°. Somit öffnet sich der Winkel des patellaren Mantelflächenabschnitts zur distalen Richtung hin. Die Fläche der zweiten Kontaktfläche kann somit größer als die Fläche der ersten Kontaktfläche sein. Damit ist die zweite Kontaktfläche zumindest teilweise an einen Querschnitt der (abgeschnittenen) Tibia angepasst. D.h. der patellare Mantelabschnitt kann eine Nase aufspannen, die über eine Grundfläche der ersten Kontaktfläche hinaussteht.

Ein Winkel, der zwischen der zweiten Kontaktfläche und dem patellaren Mantelflächenabschnitt aufgespannt ist, kann also ebenfalls ungleich 90° sein. Besonders bevorzugt ist der Winkel zwischen der zweiten Kontaktfläche und dem patellaren Mantelflächenabschnitt in dem implantierten Zustand des Kniegelenkimplantataugments kleiner als 90°.

Vorzugsweise sind zumindest drei Seiten der Mantelfläche bezüglich der ersten und / oder der zweiten Kontaktfläche angestellt. D.h. zumindest drei Seiten der Mantelfläche sind nicht senkrecht zu den Kontaktflächen ausgerichtet, sondern sind in einem Winkel ungleich 90° zu den Kontaktflächen angestellt.

Nach einem weiteren optionalen Aspekt der vorliegenden Offenbarung öffnet sich der Anstellwinkel zwischen dem patellaren Mantelflächenabschnitt und der Achse in eine kaudale / distale Richtung. D.h. der Flächeninhalt der zweiten Kontaktfläche kann vergrößert werden. Dadurch kann sichergestellt werden, dass sich (die abgeschnittene) Tibia und das Kniegelenkimplantataugment auf einer möglichst großen Fläche überschneiden und eine feste Anbindung gewährleistet wird.

Vorzugsweise ist das Kniegelenkimplantataugment annähernd u- oder hufeisenförmig ausgebildet. Der patellare Mantelflächenabschnitt kann dabei in einem Schenkel des Hufeisens positioniert sein. Das u- oder hufeisenförmige Kniegelenkimplantataugment kann zwei Schenkel aufweisen, die sich von einem Mittelabschnitt im Wesentlichen parallel in laterale Richtung erstrecken. Der patellare Mantelflächenabschnitt kann an einem der zwei Schenkel angeordnet sein. Dabei kann der patellare Mantelflächenabschnitt derart positioniert sein, dass er zu einer Außenseite des "Hufeisens" zeigt.

In dem implantierten Zustand des Kniegelenkimplantataugments kann der Mittelabschnitt (des "Hufeisens") in seitlicher Richtung des Kniegelenks zeigen. Die beiden Schenkel können sich ausgehend davon in laterale Richtung erstrecken. Somit kann ein Schenkel im implantierten Zustand patellar bzw. anterior und der andere posterior positioniert sein. Der patellare Mantelflächenabschnitt kann insbesondere an dem patellaren Schenkel des hufeisenförmigen Kniegelenkimplantataugments angeordnet sein. So kann der patellare Mantelflächenabschnitt die patellare Auflagefläche zwischen dem Kniegelenkimplantataugment und der Tibia vergrößern und es ermöglichen, das Kniegelenkimplantat ausgerichtet zur Längsachse der Tibia zu montieren.

Nach einem weiteren optionalen Aspekt der vorliegenden Offenbarung ist eine längliche Aussparung in dem (u- oder hufeisenförmigen) Kniegelenkimplantataugment dafür vorbereitet und ausgebildet, einen (Verstärkungs-)Flügel des Kniegelenkimplantats aufzunehmen und der patellare Mantelflächenabschnitt erstreckt sich im Wesentlichen parallel zu einer Längserstreckung der Aussparung. In die Aussparung des u- oder hufeisenförmigen Kniegelenkimplantataugments kann der (Verstärkungs-)Flügel eingebracht werden. Die Kombination aus der Ausrichtung des (Verstärkungs-)Flügels und der Aussparung können das Kniegelenkimplantataugment in dem implantierten Zustand positionsbestimmt an dem Kniegelenkimplantat anbringen. Somit kann die Aussparung die Position und Orientierung des Kniegelenkimplantataugments an dem Kniegelenkimplantat definieren.

Vorzugsweise hat das Kniegelenkimplantataugment in einer Seitenansicht bzw. in einem Querschnitt die Form eines Parallelogramms. Dabei können sowohl der patellare Mantelabschnitt als auch der posteriore Mantelabschnitt angestellt sein. Die beiden Mantelabschnitte können insbesondere parallel zueinander angestellt sein.

Es ist möglich, dass die beiden Mantelabschnitte jeweils bezüglich der Achse angestellt, aber nicht in dem gleichen Winkel angestellt sind.

Vorzugsweise ist zumindest ein Teil der ersten Grundfläche des Kniegelenkimplantataugments an die Form der Grundplatte des Tibiaelements angepasst. D.h. die Fläche des Kniegelenkimplantataugments, die direkt an der Grundplatte des Tibiaelements anliegt, hat zumindest zum Teil die Form der Grundstruktur. Somit gehen die beiden Elemente nahtlos ineinander über.

Nach einem weiteren optionalen Aspekt der vorliegenden Offenbarung ist ein Teil der zweiten Grundfläche des Kniegelenkimplantataugments zumindest dazu ausgebildet und angepasst, einer Außenkontur der Tibia zu entsprechen. D.h. die zweite Grundfläche kann im Wesentlichen der Form des Tibiaquerschnitts entsprechen. Somit kann die abgeschnittene Tibia ohne Absatz in das Kniegelenkimplantataugment übergehen. D.h. der Querschnitt der abgeschnittenen Tibia kann zumindest in einem Teil oder Abschnitt der zweiten Grundfläche entsprechen.

Kurzbeschreibung der Figuren
Fig. 1 zeigt ein Kniegelenkimplantat nach dem Stand der Technik;
Fig. 2 zeigt eine perspektivische Ansicht eines Tibiaelements mit einem Kniegelenkimplantataugment nach dem Stand der Technik an einer Tibia;
Fig. 3 zeigt eine Seitenansicht des Kniegelenkimplantataugments nach dem Stand der Technik;
Fig. 4 zeigt eine Seitenansicht eines Kniegelenkimplantataugments gemäß der vorliegenden Offenbarung;
Fig. 5 zeigt eine isometrische Darstellung des Kniegelenkimplantataugments gemäß der vorliegenden Offenbarung;
Fig. 6 zeigt eine Unterseite des Kniegelenkimplantataugments gemäß der vorliegenden Offenbarung; und
Fig. 7 zeigt eine Seitenansicht eines Tibiaelements mit dem Kniegelenkimplantataugment gemäß der vorliegenden Offenbarung.

### Detaillierte Beschreibung der Figuren

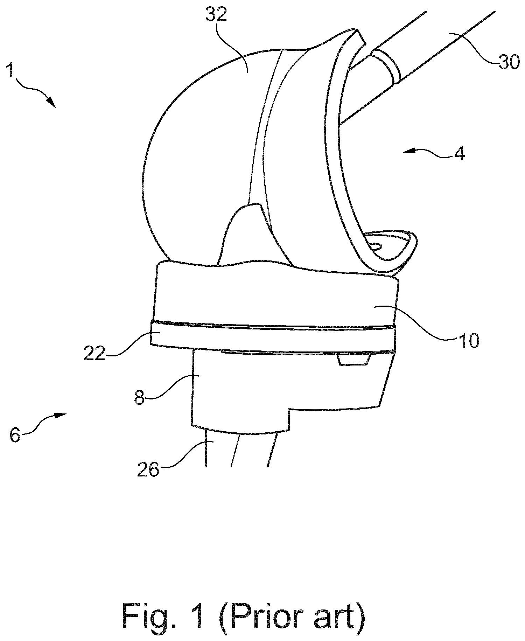

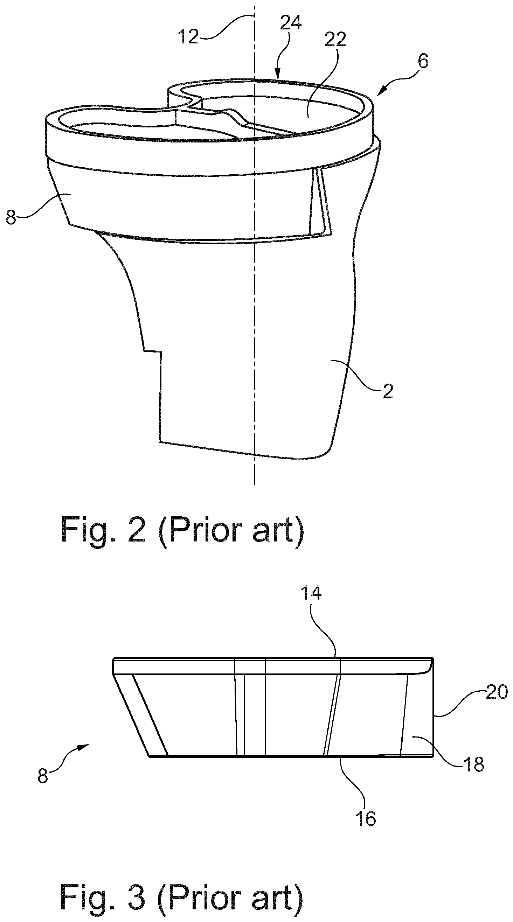

Die Figuren 1 bis 3 zeigen ein Kniegelenkimplantat oder eine Knieprothese 1 nach dem Stand der Technik. Das Kniegelenkimplantat 1 ist in einem implantierten Zustand vorzugsweise zwischen einem distalen Abschnitt des Femurs (Oberschenkelknochen) und einem proximalen Abschnitt der Tibia (Schienbeinknochen) 2 positioniert und weist ein Femurelement bzw. -implantat 4, ein Tibiaelement bzw. -implantat 6 mit einem (Kniegelenkimplantat-)Augment 8 und ein Inlay/eine Gleitschicht 10 auf. Das Inlay 10 ist zwischen dem Femurelement 4 und dem Tibiaelement 6 angeordnet und ermöglicht ein Abgleiten des Femurelements 4 relativ zu dem Tibiaelement 6.

Das Kniegelenkimplantataugment 8 ist ausgebildet, in dem implantierten Zustand zwischen dem Kniegelenkimplantat 1 und der Tibia 2 angeordnet zu sein und weist eine proximale erste Kontaktfläche 14, die ausgebildet ist, in dem implantierten Zustand des Kniegelenkimplantataugments 8 zumindest abschnittsweise flächig an dem Kniegelenkimplantat 1 aufzuliegen, und eine distale / kaudale zweite Kontaktfläche 16 auf, die ausgebildet ist, in dem implantierten Zustand zumindest abschnittsweise flächig an der Tibia 2 aufliegen zu können. Das Kniegelenkimplantataugment 8 weist ferner eine Mantelfläche 18 auf, die einen Rand der ersten Kontaktfläche 14 mit einem Rand der zweiten Kontaktfläche 16 verbindet. Die Mantelfläche 18 weist einen Mantelflächenabschnitt 20 auf, der in dem implantierten Zustand des Kniegelenkimplantataugments 8 patellar angeordnet ist.

An der ersten Kontaktfläche 14 liegt eine dünne Grundplatte 22 des Tibiaelements 6 an. Die Grundplatte 22 weist an ihrer proximalen Seite eine Aufnahmestruktur 24 für das Inlay 10 auf. An der kaudalen / distalen Seite der Grundplatte 22 erstreckt sich in distaler Richtung ein Tibiaschaft 26. Der Tibiaschaft 26 ist dafür vorbereitet und angepasst, in die Tibia 2 versenkt zu werden, um eine feste Verbindung zwischen der Tibia 2 und dem Tibiaelement 6 zu gewährleisten. Der Schaft 26 entspricht in dem implantierten Zustand des Kniegelenkimplantats 1 einer Längsachse 12 eines Markkanals (nicht dargestellt) der Tibia 2.

Wenn ein großer Teil der Tibia 2 entfernt wird, kann das dünne Tibiaelement 6 den verlorenen Knochen nicht ersetzen. Deshalb wird zusätzlich das Kniegelenkimplantataugment 8 verwendet. Die erste Kontaktfläche 14 des Kniegelenkimplantataugments 8 ist dafür vorbereitet und angepasst, an einer distalen Fläche der Grundplatte 22 anzuliegen.

An der dem Tibiaelement 6 gegenüberliegenden Seite des Inlays 10 liegt das Femurelement 4 an. Das Femurelement 4 weist dabei einen Verbindungsabschnitt 32, mit dem das Femurelement 4 mit dem Femur verbunden wird und einen Abrollabschnitt 34 auf, der auf dem Inlay 10 abrollt. Das Inlay 10 ermöglicht dem Femurelement 4 und dem Tibiaelement 6 aufeinander abzugleiten und fungiert gewissermaßen als Lager zwischen den beiden Elementen 4, 6. Der Verbindungsabschnitt 32 ist im Wesentlichen ein länglicher Schaft, der mit dem Femur verbunden wird. Der Abrollabschnitt 34 weist eine konvexe halbkreisförmige Rundung auf und ist an dem Verbindungsabschnitt 32 befestigt.

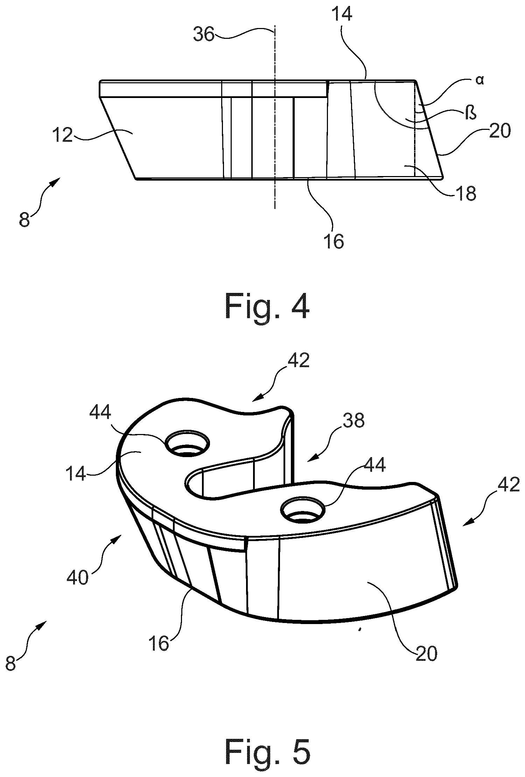

Fig. 4 zeigt eine Seitenansicht des offenbarungsgemäßen Kniegelenkimplantataugments 8. Das Kniegelenkimplantataugment 8 weist die erste Kontaktfläche 14, die zweite Kontaktfläche 16, die der ersten Kontaktfläche 14 gegenüberliegt, und die Mantelfläche 18 auf, die den Rand der ersten Kontaktfläche 14 mit dem Rand der zweiten Kontaktfläche 16 verbindet. Ferner weist das Kniegelenkimplantataugment 8 eine Achse 36 auf, die senkrecht auf der ersten Kontaktfläche 14 und / oder der zweiten Kontaktfläche 16 steht. Der Mantelflächenabschnitt 20, der in dem implantierten Zustand des Kniegelenkimplantataugments 8 patellar angeordnet ist, ist in einem Anstellwinkel α ungleich 0° bezüglich der Längsachse 12 des Markkanals der Tibia 2 angestellt.

D.h. ein Winkel α, der zwischen der Achse 36 des Kniegelenkimplantataugments 8 und dem patellaren Mantelflächenabschnitt 20 aufgespannt ist, ist vorzugsweise größer als 0°. Die Achse 36 ist dabei ein Normalenvektor auf die erste Kontaktfläche 14 bzw. die zweite Kontaktfläche 16. Der patellare Mantelflächenabschnitt 20 vergrößert so eine Auflagefläche zwischen der Tibia 2 und der zweiten Kontaktfläche 16 im Vergleich zum Stand der Technik. Damit wird eine bessere Anbindung des Kniegelenkimplantataugments 8 mit der Tibia 2 ermöglicht. Ein weiterer Abschnitt der Mantelfläche 18, der dem angestellten patellaren Mantelflächenabschnitt 20 gegenüberliegt, ist ebenfalls angestellt. Die beiden angestellten Abschnitte können die gleichen Anstellwinkel aufweisen. Ein Winkel β, der zwischen der ersten Kontaktfläche 14 und dem patellaren Mantelflächenabschnitt 20 aufgespannt ist, ist größer als 90°.

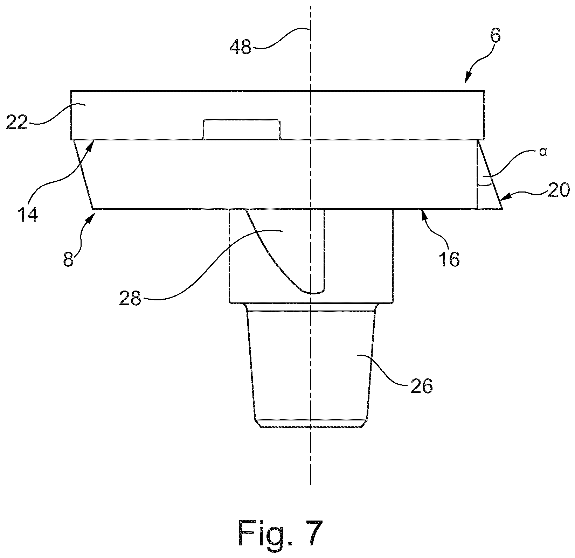

Fig. 5 zeigt eine isometrische Ansicht des Kniegelenkimplantataugments 8 gemäß der vorliegenden Offenbarung. Das Kniegelenkimplantataugment 8 weist keine durchgehende kreisförmige oder ovale Fläche oder Platte auf, sondern ist annähernd u-förmig oder hufeisenförmig ausgebildet. Das Kniegelenkimplantataugment 8 weist eine längliche Aussparung 38 auf, die dafür angepasst und vorbereitet ist, den Tibiaschaft 26 und evtl. den (Verstärkungs-)Flügel 28 (in Fig. 7 dargestellt) aufzunehmen. Das Kniegelenkimplantataugment 8 weist einen Mittelabschnitt 40 und zwei Schenkel 42 auf, die sich in dem implantierten Zustand von dem Mittelabschnitt entlang der Aussparung 38 lateral erstrecken. Der angestellte patellare Mantelflächenabschnitt 20 ist an dem Schenkel 42 ausgebildet, der in dem implantierten Zustand patellar positioniert ist.

Die Mantelfläche 18 ist, zumindest in Abschnitten, nicht senkrecht zu den beiden Kontaktflächen 14 und 16 ausgerichtet. Die Abschnitte der Mantelfläche 18, die im Mittelabschnitt 40 des u-förmigen Kniegelenkimplantataugments 8 und an einer posterioren Stirnseite positioniert sind, sind im implantierten Zustand des Kniegelenkimplantataugments 8 bezüglich der Längsachse 36 angestellt. Dabei ragt die erste Kontaktfläche 14 abschnittsweise über die zweite Kontaktfläche 16 hinaus. So ist die Form des Kniegelenkimplantataugments 8 im Wesentlichen an die Form der Tibia 2 angepasst. Das Kniegelenkimplantataugment 8 weist ferner zwei durch die Grundplatte 12 durchgehende Löcher 44 auf, durch die das Kniegelenkimplantataugment 8 mit dem Tibiaelement 6 verschraubt wird.

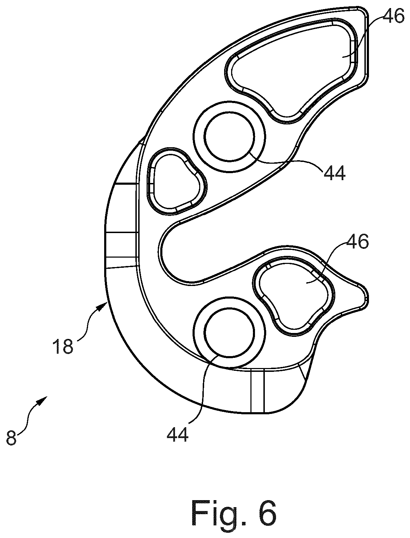

Fig. 6 zeigt eine Unterseite des Kniegelenkimplantataugments 8. Die Löcher 44 sind durch die gesamte Dicke des Kniegelenkimplantataugments 8 durchgängig und münden in der zweite Kontaktfläche 16. Die Unterseite bzw. die zweite Kontaktfläche 16 des Kniegelenkimplantataugments 8 weist eine Anzahl an Ausnehmungen 46 auf. Die Ausnehmungen 46 sind dafür angepasst, das Kniegelenkimplantataugment 8 passgenau an der Tibia 2 anzubringen. In der Unteransicht ist gezeigt, dass die Mantelfläche 18 in einem seitlichen und einem posterioren Abschnitt über die zweite Kontaktfläche 16 übersteht.

Fig. 7 zeigt eine Seitenansicht von dem Tibiaelement 6 mit dem Kniegelenkimplantataugment 8. Die erste Kontaktfläche 14 des Kniegelenkimplantataugments 8 liegt flächig an der Grundplatte 22 des Tibiaelements 6 an. Aus der zweiten Kontaktfläche 16 steht der Tibiaschaft 26 hervor. Der (Verstärkungs-)Flügel 28 verbindet den Tibiaschaft 26 mit der Grundplatte 22 und wird in der Aussparung 38 des Kniegelenkimplantataugments 8 aufgenommen. Der patellare Mantelflächenabschnitt 20 ist bezüglich einer Längsachse 48 des Tibiaschafts 26 in einem Anstellwinkel ungleich 0° angestellt.

### Bezugszeichenliste

- 1: Kniegelenkimplantat
- 2: Tibia
- 4: Femurelement
- 6: Tibiaelement
- 8: Kniegelenkimplantataugment
- 10: Inlay
- 12: Längsachse des Markkanals
- 14: erste Kontaktfläche
- 16: zweite Kontaktfläche
- 18: Mantelfläche
- 20: angestellter (patellarer) Mantelflächenabschnitt
- 22: Grundplatte
- 24: Aufnahmestruktur
- 26: Tibiaschaft
- 28: (Verstärkungs-)Flügel
- 32: Verbindungsabschnitt
- 34: Abrollabschnitt
- 36: Achse des Kniegelenkimplantataugments
- 38: Aussparung
- 40: Mittelabschnitt
- 42: Schenkel
- 44: Loch/Bohrung
- 46: Ausnehmung
- 48: Längsachse des Tibiaschafts

- α: Anstellwinkel
- β: Winkel zwischen dem patellaren Mantelflächenabschnitt und der ersten Kontaktfläche

## Patentansprüche

1. Kniegelenkimplantataugment (8), das ausgebildet ist, in einem implantierten Zustand zwischen einem Kniegelenkimplantat (1) und einer Tibia (2) angeordnet zu sein, mit
einer ersten Kontaktfläche (14), die ausgebildet ist, in dem implantierten Zustand zumindest abschnittsweise flächig an dem Kniegelenkimplantat (1) aufzuliegen,
einer der ersten Kontaktfläche (14) gegenüberliegenden zweiten Kontaktfläche (16), die ausgebildet ist, in dem implantierten Zustand zumindest abschnittsweise flächig an der Tibia (2) aufliegen zu können, und
einer Mantelfläche (18), die einen Rand der ersten Kontaktfläche (14) mit einem Rand der zweiten Kontaktfläche (16) verbindet,
**dadurch gekennzeichnet, dass**
die Mantelfläche (18) derart ausgebildet ist, dass ein Abschnitt (20) der Mantelfläche (18), welcher in dem implantierten Zustand des Kniegelenkimplantataugments (8) patellar angeordnet ist, ausgehend von der ersten Kontaktfläche (14) in einem Anstellwinkel (α) größer als 0° bezüglich einer Achse (36) in patellare Richtung angestellt ist, wobei die Achse (36) senkrecht auf der ersten Kontaktfläche (14) steht.

2. Kniegelenkimplantataugment (8) nach Anspruch 1, **dadurch gekennzeichnet, dass** die erste Kontaktfläche (14) in dem implantierten Zustand des Kniegelenkimplantataugments (8) in eine proximale Richtung zeigt.

3. Kniegelenkimplantataugment (8) nach Anspruch 1 oder 2, **dadurch gekennzeichnet, dass** ein Winkel zwischen der ersten Kontaktfläche (14) und dem patellaren Mantelflächenabschnitt (20) in dem implantierten Zustand des Kniegelenkimplantataugments (8) größer als 90° ist, wobei insbesondere eine Nase, die durch den patellaren Mantelflächenabschnitt (20) aufgespannt wird, über eine Grundfläche der ersten Kontaktfläche (14) hinaussteht.

4. Kniegelenkimplantataugment (8) nach einem der Ansprüche 1 bis 3, **dadurch gekennzeichnet, dass** ein Winkel zwischen der zweiten Kontaktfläche (16) und dem patellaren Mantelflächenabschnitt (20) in dem implantierten Zustand des Kniegelenkimplantataugments (8) kleiner als 90° ist.

5. Kniegelenkimplantataugment (8) nach einem der Ansprüche 1 bis 4, **dadurch gekennzeichnet, dass** der patellare Mantelflächenabschnitt (20) an einem Schenkel (42) des Kniegelenkimplantataugments (8), vorzugsweise an einem Schenkel (42), der in dem implantierten Zustand des Kniegelenkimplantataugments (8) patellar angeordnet ist, positioniert ist.

6. Kniegelenkimplantataugment (8) nach einem der Ansprüche 1 bis 5, **dadurch gekennzeichnet, dass** eine längliche Aussparung (38) in dem Kniegelenkimplantataugment (8) dafür vorbereitet und ausgebildet ist, einen Verstärkungsflügel (28) des Kniegelenkimplantats (1) aufzunehmen und sich der patellare Mantelflächenabschnitt (20) im Wesentlichen parallel zu einer Längserstreckung der Aussparung (38) erstreckt.

7. Kniegelenkimplantat (1) mit
einem Femurelement (4),
einem Tibiaelement (6) mit einer Grundplatte (22), einem Schaft (26), der sich von der Grundplatte (22) erstreckt und vorzugsweise einem Verstärkungsflügel (28), der sich zwischen der Grundplatte (22) und dem Schaft (26) erstreckt, und
einem Kniegelenkimplantataugment (8) nach einem der vorhergehenden Ansprüche, wobei die erste Kontaktfläche (14) zumindest abschnittsweise flächig an der Grundplatte (22) anliegt, und der Abschnitt (20) der Mantelfläche (18), welcher in dem implantierten Zustand des Kniegelenkimplantats (1) patellar angeordnet ist, in dem implantierten Zustand in einem Anstellwinkel größer als 0° bezüglich einer Längsachse (48) des Schafts (26) angestellt ist.

8. Kniegelenkimplantat (1) nach Anspruch 7, **dadurch gekennzeichnet, dass** die erste Kontaktfläche (14) zumindest abschnittsweise an eine Form der Grundplatte (22) angepasst ist.

## Claims

1. A knee joint implant augmentation (8) configured to be arranged between a knee joint implant (1) and a tibia (2) in an implanted state, comprising
a first contact surface (14) which, in the implanted state, is configured to rest at least partially flat on the knee joint implant (1),
a second contact surface (16) lying opposite the first contact surface (14) and configured to rest at least partially flat on the tibia (2) in the implanted state, and
a lateral surface (18), which connects a rim of the first contact surface (14) to a rim of the second contact surface (16),
**characterized in that**
the lateral surface (18) is configured such that a portion (20) of the lateral surface (18), which is arranged in a patellar position in the implanted state of the knee joint implant augmentation (8), is angled, starting from the first contact surface (14), at a closed angle (α) larger than 0° with respect to an axis (36) in the patellar direction, wherein the axis (36) is perpendicular to the first contact surface (14).

2. The knee joint implant augmentation (8) according to claim 1, **characterized in that** the first contact surface (14) points in a proximal direction in the implanted state of the knee joint implant augmentation (8).

3. The knee joint implant augmentation (8) according to claim 1, **characterized in that** an angle between the first contact surface (14) and the patellar lateral-surface portion (20) in the implanted state of the knee joint implant augmentation (8) is greater than 90°, wherein, in particular, a nose spanned by the patellar lateral surface portion (20) protrudes beyond a base surface of the first contact surface (14)..

4. The knee joint implant augmentation (8) according to one of claims 1 to 3,
**characterized in that** an angle between the second contact surface (16) and the patellar lateral-surface portion (20) in the implanted state of the knee joint implant augmentation (8) is less than 90°.

5. The knee joint implant augmentation (8) according to one of claims 1 to 4,
**characterized in that** the patellar lateral-surface portion (20) is positioned at a leg (42) of the knee joint implant augmentation (8), preferably at a leg (42) that is in a patellar position in the implanted state of the knee joint implant augmentation (8).

6. The knee joint implant augmentation (8) according to one of claims 1 to 5,
**characterized in that** an elongated notch (38) in the knee joint implant augmentation (8) is prepared and configured to receive a reinforcing wing (28) of the knee joint implant (1), and the patellar lateral-surface portion (20) extends substantially parallel to a longitudinal extent of the notch (38).

7. A knee joint implant (1) comprising
a femur element (4),
a tibia element (6) comprising a base plate (22), a shaft (26) extending from the base plate (22) and preferably a reinforcing wing (28) extending between the base plate (22) and the shaft (26), and
a knee joint implant augmentation (8) according to one of the preceding claims, wherein the first contact surface (14) rests at least partially flat against the base plate (22), and the portion (20) of the lateral surface (18), which, in the implanted state of the knee joint implant (1), is arranged in a patellar position, is angled in the implanted state at a closed angle larger than 0° with respect to a longitudinal axis (48) of the shaft (26).

8. The knee joint implant according to claim 7, **characterized in that** the first contact surface (14) at least partially corresponds to a shape of the base plate (22).

## Revendications

1. Augmentation d'implant articulaire du genou (8) conçue pour être disposée, à un état implanté, entre un implant articulaire du genou (1) et un tibia (2), avec
une première surface de contact (14) qui est conçue pour reposer au moins par sections à plat sur l'implant articulaire du genou (1) à l'état implanté,
une seconde surface de contact (16) opposée à la première surface de contact (14), qui est conçue pour pouvoir reposer au moins par sections à plat sur le tibia (2) à l'état implanté, et
une surface d'enveloppe (18) qui connecte un bord de la première surface de contact (14) à un bord de la seconde surface de contact (16),
**caractérisée en ce que**
la surface d'enveloppe (18) est conçue de sorte qu'une section (20) de la surface d'enveloppe (18), laquelle, à l'état implanté de l'augmentation d'implant articulaire du genou (8), est disposée de manière patellaire, est placée à partir de la première surface de contact (14) selon un angle d'attaque (a) supérieur à 0° par rapport à un axe (36) en direction patellaire, dans laquelle l'axe (36) est perpendiculaire à la première surface de contact (14).

2. Augmentation d'implant articulaire du genou (8) selon la revendication 1, **caractérisée en ce que** la première surface de contact (14) pointe dans une direction proximale à l'état implanté de l'augmentation d'implant articulaire du genou (8).

3. Augmentation d'implant articulaire du genou (8) selon la revendication 1 ou 2, **caractérisée en ce qu'**un angle entre la première surface de contact (14) et la section de surface d'enveloppe patellaire (20) à l'état implanté de l'augmentation d'implant articulaire du genou (8) est supérieur à 90°, dans laquelle en particulier un nez, qui est formé par la section de surface d'enveloppe patellaire (20), dépasse d'une surface de base de la première surface de contact (14).

4. Augmentation d'implant articulaire du genou (8) selon l'une quelconque des revendications 1 à 3, **caractérisé en ce qu'**un angle entre la seconde surface de contact (16) et la section de surface d'enveloppe patellaire (20) est inférieur à 90° à l'état implanté de l'augmentation d'implant articulaire du genou (8).

5. Augmentation d'implant articulaire du genou (8) selon l'une quelconque des revendications 1 à 4, **caractérisée en ce que** la section de surface d'enveloppe patellaire (20) est positionnée sur une branche (42) de l'augmentation d'implant articulaire du genou (8), de préférence sur une branche (42) qui, à l'état implanté de l'augmentation d'implant articulaire du genou (8), est disposée au niveau patellaire.

6. Augmentation d'implant articulaire du genou (8) selon l'une quelconque des revendications 1 à 5, **caractérisé en ce qu'**un évidement allongé (38) dans l'augmentation d'implant articulaire du genou (8) est préparé et conçu pour loger une aile de renfort (28) de l'implant articulaire du genou (1) et la section de surface d'enveloppe patellaire (20) s'étend sensiblement parallèlement à une extension longitudinale de l'évidement (38).

7. Implant articulaire du genou (1) avec
un élément fémoral (4),
un élément tibial (6) avec une plaque de base (22), une tige (26) qui s'étend à partir de la plaque de base (22) et de préférence une aile de renfort (28) qui s'étend entre la plaque de base (22) et la tige (26), et
une augmentation d'implant articulaire du genou (8) selon l'une quelconque des revendications précédentes, dans lequel la première surface de contact (14) repose au moins par sections à plat sur la plaque de base (22) et la section (20) de la surface d'enveloppe (18), laquelle, à l'état implanté de l'implant articulaire du genou (1), est disposée de manière patellaire, est placée à l'état implanté selon un angle d'attaque supérieur à 0° par rapport à un axe longitudinal (48) de la tige (26).

8. Implant articulaire du genou (1) selon la revendication 7, **caractérisé en ce que** la première surface de contact (14) est adaptée au moins par sections à une forme de la plaque de base (22).
